# EUROPEAN PATENT SPECIFICATION

(11) **EP 1 044 960 B1**
(45) Date of publication and mention of the grant of the patent: **04.12.2002**
(21) Application number: 00303079.8
(22) Date of filing: 12.04.2000
(51) Int. Cl.: C07C 209/52, C07C 211/15

(54) **Process for the preparation of 1,1,1-trifluoro-2-aminoalkanes**
Verfahren zur Herstellung von 1,1,1-Trifluoro-2-aminoalkane
Procédé pour la préparation de 1,1,1-trifluoro-2-aminoalkanes

(30) Priority: 15.04.1999 US 292756
(43) Date of publication of application: 18.10.2000
(73) Proprietor: BASF AKTIENGESELLSCHAFT, 67056 Ludwigshafen (DE)
(72) Inventor: Niedermann, Hans-Peter, 55270 Bubenheim (DE); Gutheil, Dieter, 55545 Bad Kreuznach (DE)
(74) Representative: Langfinger, Klaus Dieter, Dr.

(56) References cited:
- DICKEY J B ET AL: "FLUORINATED AMINOANTHRAQUINONE DYES" INDUSTRIAL AND ENGINEERING CHEMISTRY,US,AMERICAN CHEMICAL SOCIETY, vol. 48, no. 48, 1956, pages 209-213, XP000874162

## Description

### BACKGROUND OF THE INVENTION

The invention relates to an improved process for the preparation of 1,1,1-trifluoro-2-aminoalkanes which comprises hydrogenating the corresponding oximes.

1,1,1-trifluoro-2-aminoalkanes are useful as intermediates for the preparation of a variety of compounds which are useful as agrochemicals, pharmaceuticals or dyes. In particular, they are key intermediates in the preparation of insecticidal benzamides as disclosed for example by DE 36 11 193 and of fungicidal 7-(1,1,1-trifluoroalk-2-ylamino)-6-(halophenyl)-triazolopyrimidines which are described for example in PCT/US98/05615.

J.B. Dickey et al., Ind. Eng. Chem. **98**, 1956, 209-213 disclose a method for the preparation of 1,1,1-trifluoro-2-aminopropane by the hydrogenation of the corresponding oxime in a rocking autoclave at a pressure of 150 bar (2.000 p.s.i.) in the presence of Raney Nickel using ether as diluent. However, only 30 % of the desired product are obtainable according to this method.

Therefore, the method known from the art is not applicable for large scale production, due to its low yields.

### SUMMARY OF THE INVENTION

Surprisingly, the 1,1,1-trifluoro-2-aminoalkanes of formula I 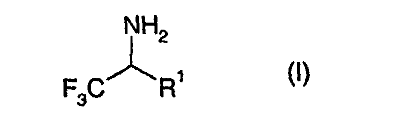 wherein
R¹ represents an optionally substituted alkyl group;
can be obtained in high yields by hydrogenation of corresponding oximes of formula II 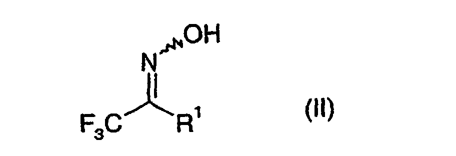 wherein R¹ has the meaning given above and the winding line indicates that the hydroxy group may be in the (E)- or (Z)-position with respect to the trifluoromethyl group,
in the presence of Raney nickel and a diluent;
when the reaction is carried out in a diluent selected from an alkanol, a cyclic ether and an aromatic hydrocarbon.

It is, therefore, an object of the present invention to provide an efficient improved process for the preparation of 1,1,1-trifluoro-2-aminoalkanes of formula I.

Other objects and advantages of the present invention will be apparent to those skilled in the art from the following description and the appended claims.

### DETAILED DESCRIPTION OF THE PREFERRED EMBODIMENTS

The invention relates to an improved process for the preparation of the compounds of formula I by the hydrogenation of the oximes of formula II the improvement wherein is that the hyrogenation is carried out in a diluent selected from an alkanol, a cyclic ether and an aromatic hydrocarbon.

In general terms, unless otherwise stated herein, the term alkyl as used herein with respect to a radical or moiety or part thereof e.g alkoxy refer to a straight or branched chain radical or moiety. As a rule, such radicals have up to 10, in particular up to 6 carbon atoms. Suitably an alkyl or alkoxy moiety has from 1 to 6 carbon atoms, preferably from 1 to 3 carbon atoms. A particularly preferred alkyl moiety is the methyl group.

Suitable alkanols are straight chained or branched alkanols with up to 6 carbon atoms, preferably 1 to 4 carbon atoms. Most preferred are methanol, ethanol and isopropanol, in particular methanol.

Suitable cyclic ethers are cyclic ethers having 4 to 6 carbon atoms and 1 or 2 non-adjacent oxygen atoms, in particular tetrahydrofuran and dioxane.

Suitable aromatic hydrocarbons are aromatic hydrocarbons having 6 to 9 carbon atoms, in particular benzene, toluene or xylene.

A preferred embodiment of the present invention is a process wherein one or more (e.g all) of the following apply:
- 1 part of the oxime of formula II is diluted with 5 to 15 parts, preferably 7.5 to 12.5 parts, in particular 8 to 10 parts of methanol, tetrahydrofuran or toluene; and/or
- Raney nickel is used in moist form, in particular moisturized with methanol; and/or
- the mixture consisting of the oxime of formula II, the diluent and Raney nickel is kept under a hydrogen atmosphere at temperatures between 50 °C and 150 °C, preferably between 75 °C and 120 °C, in particular between 80 °C and 110 °C; most preferred at about 90 °C, and/or
- the mixture consisting of the oxime of formula II, the diluent and Raney nickel is kept under a hydrogen atmosphere for 1 to 5 hours, preferably 2 to 4 hours, in particular about 3 hours; and/or
- the hydrogenation is carried out at a pressure of 25 to 100 bar, preferably 30 to 80 bar, in particular 35 to 70 bar most preferred at a pressure of about 50 bar; and/or
- the reaction mixture obtained from the hydrogenation is filtered and the resulting filtrate is acidified with a mineral acid, preferably hydrochloric acid, to obtain the corresponding 1,1,1 -trifluoro-alk-2-yl ammonium salt; and/or
- the 1,1,1-trifluoro-alk-2-yl ammonium salt is separated and treated with a base, preferably with an alkali hydroxide, in particular sodium hydroxide to obtain the compound of formula I; and/or
- R¹ represents a C₁₋₄ alkyl group being optionally substituted by one or more halogen atoms or an alkoxycarbonyl or hydroxycarbonyl group, in particular wherein R¹ represents a methyl group.

The compound of formula II is preferably 1,1,1-trifluoropropanone oxime or ethyl 1,1,1-trifluoroacetylacetate oxime, which can be prepared by a condensation reaction between commercially available 1,1,1-trifluoropropanone or ethyl 1,1,1-trifluoroacetylacetate and hydroxylamine. This reaction is preferably carried out in the presence of a dehydration agent such as molecular sieves, TiCl₄ or sodium acetate as disclosed for example by J.B. Dickey et al., loc. cit..

The crude product obtained can be purified according to standard methods for example by distillation or chromatographic methods.

However, the crude product obtained according to the process of this invention is as a rule pure enough to be used as intermediate without further purification.

In a particularly preferred embodiment of the process according to this invention a mixture of the oxime of formula I (3 parts), the diluent, preferably methanol (25 to 35 parts) and Raney nickel (preferably moisturized with methanol; 1 to 1.5 parts) is hydrogenated in an autoclave at a pressure of about 50 bar and a temperature of about 90 °C until the hydrogen uptake ceases. The reaction mixture is cooled, filtered and treated with a mineral acid, in particular hydrochloric acid, and evaporated. The residue is treated with sodium hydroxide and the obtained amine of formula I is distilled.

In order to facilitate a further understanding of the invention, the following illustrative examples are presented. The invention is not limited to the specific embodiments described or illustrated, but encompasses the full scope of the appended claims.

### Example 1

### Preparation of 1,1,1-trifluoropropanone oxime

3720 g of hydroxylamine hydrochloride were added to a solution of 5271 g of sodium acetate in 21 I water. Subsequently, 3000 g of 1,1,1-trifluoroacetone were dosed within 45 minutes at a temperature range of -5°C - +8°C. The reaction mixture was stirred at room temperature for 3 days. After the stirrer has been switched off the phases separated. The crude oxime was diluted with a solution of sodium carbonate (700 g) in 5 I water. The oxime was separated and dried to yield 2912 g of the crude product which was used for the preparation of 2-amino-1,1,1-trifluoropropylamine without further purification.

### Example 2

### Preparation of 2-amino-1,1,1-trifluoropropylamine hydrochloride

An autoclave was charged with 2986 g of 1,1,1 -trifluoropropanone oxime obtained in Example 1, 1 kg of Raney nickel (moisturized with methanol) and 30 of methanol. The autoclave was pressurized with 1400 of hydrogen at a pressure of 50 bar and heated to 90°C. After a hydrogenation time of 2.5 h and a consumption of 64.5 bar the reaction mixture was cooled to room temperature and filtered. The filtrate was cooled down to 0°C and acidified with concentrated hydrochloric acid. The acidified solution was evaporated to dryness and the precipitate washed with diethylether and dried to yield 2974.3 g (86 %) of 2-amino-1,1,1-trifluoropropane hydrochloride having a melting point of 254-256 °C.

### Example 3

### Preparation of 2-amino-1,1,1-trifluoropropylamine

2974.3 g 2-amino-1,1,1-trifluoropropane hydrochloride obtained in Example 2 was placed in a three necked flask with stirrer, dropping funnel and descending condenser. The oil bath was heated up to 90°C and an aqueous sodium hydroxide solution (40 %) was added to the hydrochloride. The liberated amine distilled off and 2456.8 g (98 %) of the product were collected having a boiling point of 46-47 °C.

### Examples 4 to 7

### Preparation of 2-amino-1,1,1-trifluoropropylamine hydrochloride

An autoclave was charged with 12.7 g of the oxime obtained in Example 1. 6 g of Raney nickel and 130 ml of the diluent indicated in Table I. The autoclave was pressurized with 6l of hydrogen at the indicated pressure and heated to 90°C. After a hydrogenation time of 1.5 hours the reaction mixture was cooled to room temperature and filtered. The filtrate was cooled down to 0°C and acidified with concentrated hydrochloric acid. The acidified solution was evaporated to dryness and the precipitate washed with diethylether and dried to obtain 2-amino-1,1,1-trifluoropropane hydrochloride with the yields shown in Table I.

**Table I**

| **Example** | **diluent** | **pressure (bar)** | **Yield (%)** |
|---|---|---|---|
| **4** | methanol | 50 | 73.5 |
| **5** | toluene | 50 | 60.2 |
| **6** | THF | 50 | 63.5 |
| **7** | ethanol | 50 | 78.0 |
| **comparison** | diethylether | 140 | 30.0 |

## Claims

1. A process for the preparation of a 1,1,1-trifluoro-2-aminoalkane of formula I 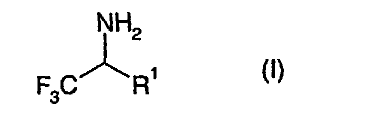 wherein
R¹ represents an optionally substituted alkyl group;
which comprises hydrogenating the corresponding oxime of formula II 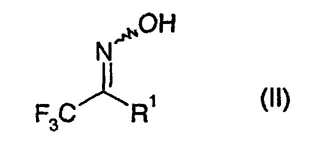 wherein R¹ has the meaning given above and the winding line indicates that the hydroxy group may be in the (E)- or (Z)-position with respect to the trifluoromethyl group,
in the presence of Raney nickel and a diluent;
**characterised in that** the reaction is carried out in a diluent selected from an alkanol, a cyclic ether and an aromatic hydrocarbon.

2. A process according to claim 1, wherein 1 part of the oxime of formula II is diluted with 5 to 15 parts of methanol, tetrahydrofuran or toluene.

3. A process according to claim 1 or claim 2, wherein Raney nickel is used in moist form.

4. A process according to claim 3, wherein Raney nickel is moisturized with methanol.

5. A process according to any one of claims 1 to 4 wherein the mixture consisting of the oxime of formula II, the diluent and Raney nickel is kept under a hydrogen atmosphere at temperatures between 50 °C and 150 °C.

6. A process according to any one of claims 1 to 5 wherein the mixture consisting of the oxime of formula II, the diluent and Raney nickel is kept under a hydrogen atmosphere for 1 to 5 hours.

7. A process according to any one of claims 1 to 6 wherein the hydrogenation is carried out at a pressure of 25 to 100 bar.

8. A process according to any one of claims 1 to 7 wherein the reaction mixture obtained from the hydrogenation is filtered and the resulting filtrate is acidified with a mineral acid to obtain the corresponding 1,1,1-trifluoro-alk-2-yl ammonium salt.

9. A process according to any one of claims 1 to 8 wherein the 1,1,1-trifluoro-alk-2-yl ammonium salt is separated and treated with a base to obtain the compound of formula I.

10. A process according to any one of claims 1 to 9 wherein R¹ represents a C₁₋₄ alkyl group being optionally substituted by one or more halogen atoms or an alkoxycarbonyl group.

11. A process according to claim 10, wherein R¹ represents a methyl group.

## Patentansprüche

1. Verfahren zur Herstellung eines 1,1,1-Trifluor-2-aminoalkans der Formel I 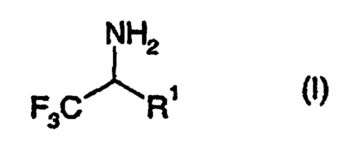 worin
R¹ für eine gegebenenfalls substituierte Alkylgruppe steht;
bei dem man das entsprechende Oxim der Formel II 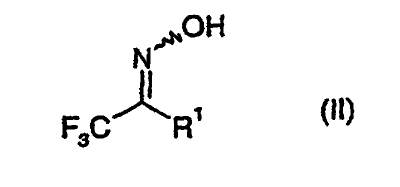 worin R¹ die angegebene Bedeutung besitzt und die Schlangenlinie anzeigt, daß die Hydroxylgruppe in (E)- oder (Z)-Stellung zur Trifluormethylgruppe stehen kann,
in Gegenwart von Raney-Nickel und einem Verdünnungsmittel hydriert;
**dadurch gekennzeichnet, daß** man die Umsetzung in einem unter einem Alkanol, einem cyclischen Ether und einem aromatischen Kohlenwasserstoff ausgewählten Verdünnungsmittel durchführt.

2. Verfahren nach Anspruch 1, bei dem man 1 Teil des Oxims der Formel II mit 5 bis 15 Teilen Methanol, Tetrahydrofuran oder Toluol verdünnt.

3. Verfahren nach Anspruch 1 oder 2, bei dem man Raney-Nickel in feuchter Form verwendet.

4. Verfahren nach Anspruch 3, bei dem das Raney-Nickel mit Methanol angefeuchtet ist.

5. Verfahren nach einem der Ansprüche 1 bis 4, bei dem man die Mischung aus dem Oxim der Formel II, dem Verdünnungsmittel und Raney-Nickel unter einer Wasserstoffatmosphäre bei Temperaturen zwischen 50°C und 150°C hält.

6. Verfahren nach einem der Ansprüche 1 bis 5, bei dem man die Mischung aus dem Oxim der Formel II, dem Verdünnungsmittel und Raney-Nickel 1 bis 5 Stunden unter einer Wasserstoffatmosphäre hält.

7. Verfahren nach einem der Ansprüche 1 bis 6, bei dem man die Hydrierung bei einem Druck von 25 bis 100 bar durchführt.

8. Verfahren nach einem der Ansprüche 1 bis 7, bei dem man die bei der Hydrierung anfallende Reaktionsmischung filtriert und das erhaltene Filtrat mit einer Mineralsäure ansäuert, wobei man das entsprechende 1,1,1-Trifluor-alk-2-ylammoniumsalz erhält.

9. Verfahren nach einem der Ansprüche 1 bis 8, bei dem man das 1,1,1-Trifluor-alk-2-ylammoniumsalz abtrennt und mit einer Base behandelt, wobei man die Verbindung der Formel I erhält.

10. Verfahren nach einem der Ansprüche 1 bis 9, bei dem R¹ für eine gegebenenfalls durch ein oder mehrere Halogenatome oder eine Alkoxycarbonylgruppe substituierte C₁₋₄-Alkylgruppe steht.

11. Verfahren nach Anspruch 10, bei dem R¹ für eine Methylgruppe steht.

## Revendications

1. Procédé pour la préparation d'un 1,1,1-trifluoro-2-aminoalcane de formule I 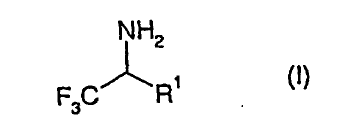 dans laquelle
R¹ représente un groupe alkyle facultativement substitué ; qui comprend l'hydrogénation de l'oxime correspondante de formule II 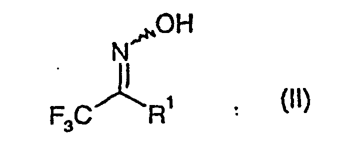 dans laquelle R¹ est tel que défini ci-dessus et la ligne brisée indique que le groupe hydroxyle peut être en position (E) ou (Z) par rapport au groupe trifluorométhyle,
en présence de nickel de Raney et d'un diluant ;
**caractérisé en ce que** la réaction est conduite dans un diluant choisi parmi un alcanol, un éther cyclique et un hydrocarbure aromatique.

2. Procédé selon la revendication 1, dans lequel 1 partie de l'oxime est formule II est diluée avec 5 à 15 parties de méthanol, de tétrahydrofuranne ou de toluène.

3. Procédé selon la revendication 1 ou la revendication 2, dans lequel le nickel de Raney est utilisé sous forme humide.

4. Procédé selon la revendication 3, dans lequel le nickel de Raney est humidifié avec du méthanol.

5. Procédé selon l'une quelconque des revendications 1 à 4, dans lequel le mélange formé par l'oxime de formule II, le diluant et le nickel de Raney est maintenu sous une atmosphère d'hydrogène à des températures comprises entre 50°C et 150°C.

6. Procédé selon l'une quelconque des revendications 1 à 5, dans lequel le mélange formé par l'oxime de formule II, le diluant et le nickel de Raney est maintenu sous une atmosphère d'hydrogène pendant 1 à 5 heures.

7. Procédé selon l'une quelconque des revendications 1 à 6, dans lequel l'hydrogénation est conduite à une pression de 25 à 100 bars.

8. Procédé selon l'une quelconque des revendications 1 à 7, dans lequel le mélange réactionnel obtenu par l'hydrogénation est filtré et le filtrat résultant est acidifié avec un acide minéral pour donner le sel de 1,1,1-trifluoroalk-2-ylammonium correspondant.

9. Procédé selon l'une quelconque des revendications 1 à 8, dans lequel le sel de 1,1,1-trifluoroalk-2-ylammonium est séparé et traité avec une base pour donner le composé de formule I.

10. Procédé selon l'une quelconque des revendications 1 à 9, dans lequel R¹ représente un groupe alkyle en C₁-C₄ facultativement substitué par un ou plusieurs atomes d'halogène ou un groupe alcoxycarbonyle.

11. Procédé selon la revendication 10, dans lequel R¹ représente un groupe méthyle.
